# EUROPEAN PATENT SPECIFICATION

(11) **EP 2 440 180 B1**
(45) Date of publication and mention of the grant of the patent: **27.08.2014**
(21) Application number: 10720981.9
(22) Date of filing: 27.05.2010
(51) Int. Cl.: A61K 9/08, A61K 47/12, A61K 38/25, A61K 9/00

(54) **IPAMORELIN DIACETATE INJECTION AND INFUSION SOLUTIONS**
INJEKTIONS- UND INFUSIONSLÖSUNGEN AUS IPAMORELINDIACETAT
SOLUTIONS D'INJECTION ET D'INFUSION DE DIACÉTATE D'IPAMORÉLINE

(30) Priority: 12.06.2009 US 186595 P
(43) Date of publication of application: 18.04.2012
(73) Proprietor: Helsinn Healthcare S.A., 6912 Lugano / Pazzallo (CH)
(72) Inventor: GARCIA RUBIO, Silvina, Princeton, NJ 08540 (US); BEIDLER, Daniel, Poway, CA 92064 (US); WEIBEL, Helle, 3400 Hillerod (DK); BRAGLIA, Riccardo, 6913 Lugano-Carabbia (CH)
(74) Representative: Zardi, Marco
(86) International application number: PCT/US2010/036365
(87) International publication number: WO 2010/144265

(56) References cited:
- WO-A1-00/74702
- WO-A1-2008/124183
- WO-A1-2009/082426
- WO-A1-2010/099522
- US-A- 5 767 085

## Description

### RELATION TO PRIOR APPLICATIONS

This application claims priority to U.S. Provisional Application 61/186,595, filed June 12, 2009.

### FIELD OF THE INVENTION

The present invention is in the field of ipamorelin chemistry, and relates particularly to methods of solubilizing ipamorelin as the diacetate salt, preferably in the presence of excess acidic residues for optimum stability and solubility in injection and infusion solutions.

### BACKGROUND OF THE INVENTION

Ipamorelin is a selective growth hormone secretagogue first synthesized by researchers at Novo Nordisk in the mid-1990s, and is described in EP 0736039 B1. The molecule is chemically defined as α-Methylalanine-L-histidine-D-β-(2-naphthyl)-alanine-D-phenylalanine-L-lysinamide or H-Aib-His-β-(2-naphthyl)-D-Ala-D-Phe-Lys-NH₂, and has the following chemical structure:

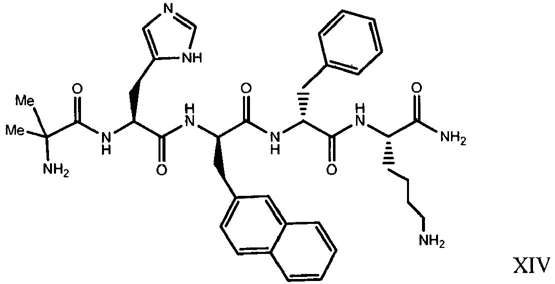

The molecule is reportedly a white amorphous powder isolated as a trifluoroacetate salt, and has a molecular weight (free base) of approximately 711.9 g/mole. Injection solutions of ipamorelin trifluoroacetate, dissolved in saline containing porcine serum albumin, are described in Raun et al., Eur. INL, ENDOCR, (1998) 139:552-561. EP 0736039 B1 mentions numerous acid addition salts of compounds structurally related to ipamorelin, but makes no mention of ways to improve the solubility of ipamorelin in injection solutions, while improving the molecule's stability.

US5767085 discloses a number of compounds, including Ipamorelin, as growth hormone secretagogues. WO0074702 discloses combinations of tyreothropin releasing hormone and a peptide drug, e.g. ipamorelin or pharmaceutically acceptable salts thereof, for the treatment of catabolic states, WO2008/124183 discloses the use of growth hormone secretagogues, inter alia ipamorelin, for treating emesis; in one example the tested drug is solubilized with acetic acid, obtaining a preparation with pH 7.5±0.2.

### SUMMARY OF THE INTENTION

The present invention provides a solution of ipamorelin solubilized by two molar equivalents of acetic acid (hereinafter referred to as "ipamorelin diacetate"), useful as finished injection and infusion solutions, methods of making the finished solutions, and novel intermediate and raw materials used in the methods, which do not lead to excessive instability, precipitation or degradation of the ipamorelin. Thus, in a first principal embodiment the invention provides novel injection and infusion solutions of ipamorelin diacetate comprising:
a) ipamorelin solubilized by approximately two molar equivalents of acetic acid (ipamorelin diacetate), in an amount of from 0.001 to 20% based on the weight of the free base of ipamorelin;
b) a molar excess of acid, preferably acetic acid, in an amount sufficient to give a pH of at least 3 and less than 7;
c) optionally from 0.1 to 30% of one or more formulation aids; and
d) water q.s. to 100%; all percentages based on the total weight of the solution.

The present invention also relates to a method of producing injection and infusion solutions of ipamorelin diacetate *in situ,* at a concentration of ipamorelin of from 0,001 to 20%, without isolating the diacetate salt after formation, comprising:
a) mixing ipamorelin free base and acetic acid at a molar ratio of approximately 1:2 to form an aqueous solution of ipamorelin diacetate;
b) mixing said ipamorelin diacetate with from 0,1 to 30% of one or more formulation aids, optionally including one or more excess acids, without isolating said ipamorelin diacetate in solid form; and
c) optionally mixing said ipamorelin diacetate with additional water so that the final concentration of ipamorelin is from 0.001 to 20%, based on the total weight of the solution and the weight of the free base of ipamorelin.
In this context, the term "mixing" is given a broad meaning, so that steps (a), (b) and (c) can occur simultaneously, sequentially or in any order. In a particularly preferred embodiment, the one or more formulation aids comprises a tonicifying effective amount of one or more tonicity agents. In another preferred embodiment, the one or more formulation aids comprises a pH adjusting agent, in an amount sufficient to yield a predetermined pH.

In yet another embodiment, the invention provides ipamorelin diacetate. Still further, the invention relates to intermediates used in the manufacture of the pharmaceutical solutions of the present invention. Thus, in another embodiment the invention provides an intermediate ipamorelin solution solubilized by acetic acid having an ipamorelin:acid molar ratio of about 1:2.

Compared with previously known injection solutions of ipamorelin, the solutions according to the invention have the advantage of improved stability when stored for prolonged periods of time. In like manner, the methods of production, and intermediates and raw materials used in the methods, ensure the stability of ipamorelin during the product's shelf life.

Additional embodiments and advantages of the invention will be set forth in part in the description which follows, and in part will be obvious from the description, or may be learned by practice of the invention. The embodiments and advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### DESCRIPTION OF THE FIGURES

The accompanying drawings, which are incorporated in and constitute a part of this specification, illustrate several embodiments of the invention and together with the description serve to explain the principles of the invention.
FIGURE 1 is a two axis graph depicting the effect of pH on the solubility of ipamorelin free base.
FIGURE 2 is an X-ray diffraction pattern of ipamorelin free base, dihydrate crystalline form, before and after storage at 98% relative humidity for twelve days.
FIGURE 3 is a hot stage X-ray diffraction pattern of ipamorelin free base, anhydrous crystalline form A.

### DETAILED DESCRIPTION OF THE INVENTION

The present invention may be understood more readily by reference to the following detailed description of preferred embodiments of the invention and the Examples included therein.

### Definitions and Use of Terms

As used in this specification and in the claims which follow, the singular forms "a," "an" and "the" include plural referents unless the context clearly dictates otherwise. Thus, for example, reference to "an ingredient" includes mixtures of ingredients, reference to "an active pharmaceutical agent" includes more than one active pharmaceutical agent, and the like.

Unless otherwise specified herein, percentages given herein are weight percentages, and the weight or percentage of ipamorelin or its salts is given based on the weight of the free base of ipamorelin, calculated on an anhydrous basis without taking into account any waters of hydration.

"Pharmaceutically acceptable" means that which is useful in preparing a pharmaceutical composition that is generally safe, non-toxic and neither biologically nor otherwise undesirable and includes that which is acceptable for veterinary use as well as human pharmaceutical use.

### Discussion

As noted herein, the present invention provides injection and infusion liquid solutions of ipamorelin in finished form, ready for administration. The invention also provides methods of making the finished solutions, and novel intermediates and raw materials used in the methods, which do not lead to excessive degradation of the ipamorelin. The injection and infusion solutions according to the invention contain the active substance ipamorelin in concentrations of from 0.001 to 20%, preferably from 0.005 to 10%, and most preferably from 0.01 to 1%. For subcutaneous formulations, an especially preferred concentration is from 0.1 to 10%. Consistent with the remainder of this document, these percentages are based on the total weight of the solution, and the weight of the free base of ipamorelin.

While the sequence of mixing can vary, the ipamorelin is preferably first solubilized in an aqueous solution of acetic acid at a molar ratio of approximately 1:2 (ipamorelin:acetate), thereby giving what is referred to herein as the diacetate salt of ipamorelin, or ipamorelin diacetate. The concentration of ipamorelin in this intermediate aqueous solution preferably ranges from 0.1 to 100 mg/ml, or from 0.5 to 20 mg/ml.

The ipamorelin diacetate is further stabilized by the presence of a molar excess of a pharmaceutically acceptable acid. The molar excess is preferably adequate to yield a solution having a pH of at least 3 or 4 and less than 7, preferably from 3 to 6.5, 4 to 6.5, 4.5 to 6.5, 3.5 to 4.5, 4.5 to 5.5, 5.5 to 6.5, 3 to 4, 4 to 5, 5 to 6, or 6 to 7. Although various acids can be used to stabilize the ipamorelin diacetate, including citric acid, hydrochloric acid, methanesulphonic acid, ethanesulphonic acid, propionic acid, succinic acid, glutaric acid, ascorbic acid, phosphoric acid, tartaric acid, lactic acid, and mixtures thereof, in a preferred embodiment the ipamorelin diacetate is stabilized by an excess of acetic acid.

The acetic acid is preferably present in solution at a molar excess of from 0.1 to 30, relative to the ipamorelin diacetate. Thus, for example, if the solution contains 1 mol of ipamorelin diacetate, the solution will further contain from 0.1 to 30 moles of excess acetic acid. In preferred embodiments, the molar excess ranges from 1 to 10, from 2 to 5, or from 5 to 8.

While it is preferred to work exclusively with water as the diluent for the final and intermediate solutions, other liquid diluents can be combined with the water for injections, including ethanol, glycerol, propylene glycol, polyethylene glycol and triethylene glycol. In a preferred embodiment, a pH buffering system is created in the final formulation by the inclusion of an alkaline salt of a suitable organic acid, preferably corresponding to the excess acid component (i.e. sodium acetate or sodium citrate). Suitable concentrations of the buffering system range from about 5 to 20 or 40 milliMolar. Thus, for example, if an acetate buffering system is employed, the excess acetate ions in solution, excluding any acetate from the ipamorelin diacetate, would range from 5 to 20 or 40 milliMolar.

The osmolality of the aqueous solutions is preferably 200 to 900 mOsmol/kg, more preferably 260 to 390 mOsmol/kg. The solution can be adapted to isotonic conditions by the addition of tonicifying agent selected from NaCl, glucose, fructose, glycerol, sorbitol, mannitol, sucrose or xylitol, or a mixture of these substances.

It is also possible to use formulation aids such as thickeners (e.g. inter alia methyl cellulose, hydroxyethyl cellulose, hydroxypropyl cellulose, sodium carboxymethyl cellulose, polyvinylpyrrolidone and gelatin), absorbents, light stabilizers, crystallization retarders, complexing agents (e.g. inter alia NaEDTA, phosphates, nitrates, acetates and citrates), antioxidants (inter alia ascorbic acid, sulphite compounds, L-cysteine, thiodipropionic acid, thiolactic acid, monothioglycerol and propyl gallate) and preservatives (inter alia PHB esters, phenol and derivatives, chlorobutanol, benzyl alcohol, ethanol, butanol, butane-1,3-diol, chlorhexidine salts, benzoic acid and salts, and sorbic acid).

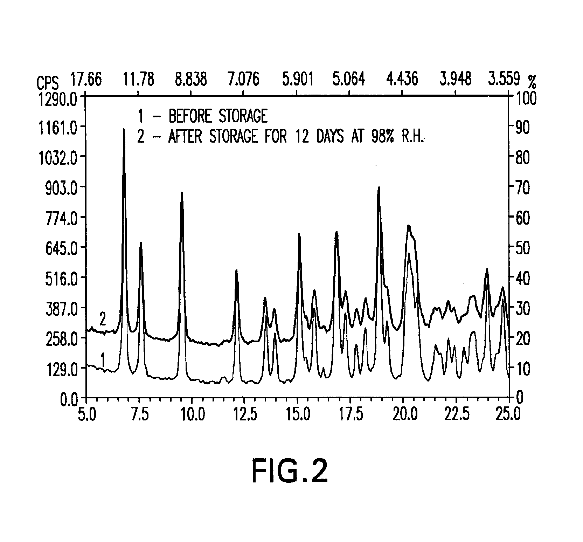

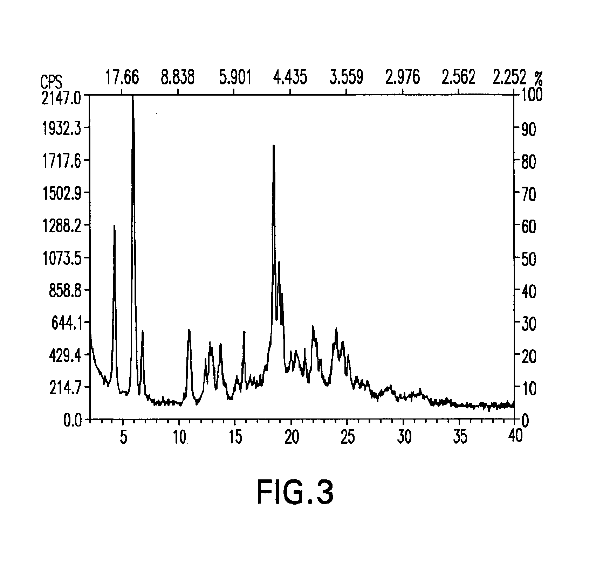

The injection or infusion solutions according to the invention are preferably prepared using the isolated free base of ipamorelin, having a high degree of purity. On an anhydrous basis, excluding water from the calculation, the ipamorelin preferably has a purity of greater than 90, 95 or even 98 wt.%. While any crystalline or amorphous form of ipamorelin will work, the starting material is preferably a dihydrate crystalline form of ipamorelin, as opposed to the amorphous form or crystalline forms A and B, as illustrated in Figures 2 and 3. Alternatively, the ipamorelin can be a crystalline form having from 1.5 to 2.0 or 2.5 moles of water per mole of ipamorelin. These hydrated forms of ipamorelin are preferably obtained by precipitation of the free base from water/methanol. The waters of hydration are lost upon heating to about 110-110° C, as revealed by differential scanning calorimetry (DSC). Anhydrous form A can be obtained via precipitation of the free base in acetonitrile. Anhydrous form B is enantiotropic with form A, and is obtained when form A is heated above about 110 °C.

The salts are then preferably prepared directly in solution by combining the ipamorelin with the amount of acetic acid required for solubilization and salt formation. The dissolution can be accelerated by working at temperatures of between 30 and 60° C, and the solutions can be prepared under nitrogen gas to minimize oxidation.

The final solution may then be prepared with or without first isolating the ipamorelin salt in a solid form. If the diacetate salt is isolated, it is preferably isolated and stored in an amorphous state, preferably at a purity exceeding 90, 95 or 98wt.% (on an anhydrous basis excluding water from the calculation). Once the acetic acid salt of ipamorelin is formed, it may be combined with other formulation aids, or water for injection to the desired concentration in the final formulation.

When the solution is prepared without first isolating the ipamorelin salt, the solution will lack any solvent residue that commonly remains when a salt is precipitated from a solvent. Thus, in another embodiment, the invention provides the solutions of the present invention in the absence of solvent residue used for the precipitation of the ipamorelin salt. While other methods can be used to eliminate these residues, this *in situ* method has proven especially beneficial. The absence of solvent residue can be proven by the lack of residue below detectable limits, using well-known methods for solvent detection and quantification.

In this way it is possible to prepare either ready-to-use solutions of the active substance filled into suitable containers, e.g. into ampoules, injection vials, infusion bottles, syringes, or precursors suitable for the preparation of such solutions, e.g. concentrates or lyophilized. The containers into which the preparations are filled can be made either of glass or of plastic, it being possible for the container materials to include substances which give the contents a particular protection, e.g. protection from light or oxygen. Once prepared, the solution may be administered directly, without further reconstitution, via injection or infusion to a patient in need of treatment by ipamorelin.

### EXAMPLES

The preparation of the solutions in the following Examples can be carried out in batch vessels with or without a heat transfer jacket. When using a non-heatable vessel, preheated water can be used if necessary. In general, the bulk of the solvent is introduced into the vessel and the individual components are dissolved therein, although it is also possible to add the solvent to the solids.

The other constituents are subsequently dissolved in or incorporated into the preparation before or after cooling, with stirring. After making-up with the remainder of the solvent, the formulation can be sterile-filtered through suitable bacteria-retaining filters and/or heat -sterilized.

### EXAMPLE 1: Exemplary Drug Products and Stability Thereof

### PREPARATION OF STOCK SOLUTIONS

### 3 L of 5.0 mg/mL Ipamorelin Stock

Three preparations of 1000 mL were prepared with 5.01256, 5.01767, and 5.01252 gm of Ipamorelin (API) respectively. Each solution contained 800 µL glacial acetic acid and was QS'd with Milli-Q water. The three solutions were combined into a single vessel and thoroughly mixed to provide the final Ipamorelin stock solution.

### 12 L of 18 mg/mL NaCl Stock

4000 mL was prepared by adding 72.0 gm of NaCl with Milli-Q water and mixed until dissolved.

### 100 mM sodium acetate, pH 5

1000 mL was prepared with 1.71 mL of glacial acetic acid and 9.574 gm of sodium acetate. Final pH of buffer was pH 5.41.

### 100 mM sodium acetate, pH 6

1000 mL was prepared with 0.14 mL of glacial acetic acid and 13.262 gm of sodium acetate. Final pH of buffer was pH 6.26.

### 100 mM sodium citrate, pH 5

1000 mL was prepared with 8.614 gm of citric acid monohydrate and 17.352 gm of sodium citrate. Final pH of buffer was pH 4.99.

### 100 mM sodium citrate, pH 6

1000 mL was prepared with 3.992 gm of citric acid monohydrate and 24.410 gm of sodium citrate. Final pH of buffer was pH 5.58.

### 100 mM sodium citrate, pH 7

1000 mL was prepared with 29.410 gm of sodium citrate with an initial pH 8.81 and was adjusted to pH 7.33 with 0.1N Hydrochloric acid.

### 100 mM sodium phosphate, 20 mM EDTA, pH6

100 mL was prepared with 5.382 gm of sodium phosphate monobasic monohydrate, 16,352 gm of sodium citrate and 7.445 gm of disodium ethylenediaminetetraacetate. Final pH of buffer was pH 5.98.

### PREPARATION OF DRUG PRODUCTS

### Control 0.5 mg/mL Ipamorelin in 2x equivalents of acetic acid pH 7.5, and 9 mg/mL

1000 mL was prepared with 500 mL of 2x sodium chloride, 100 mL of 10x Ipamorelin (API) stock solution with an initial pH of 6.95 was adjusted to pH 7.53 using 6 drops of IN sodium hydroxide. Mixed by inversion, 15x and filtered with Durapore 0.22 µm Millipore filter. Dispensed 10 mL aliquots with an Eppendorf repeat pipetter and Biopur 50 mL Combitip.

### DP#1: 0.5 mg/mL Ipamorelin, 10 mM sodium acetate, pH 5, and 9mg/mL NaCl

900 mL of the formulation was prepared by mixing 100 mL of 10x sodium acetate pH ~5 stock solution with 500 mL NaCl stock solution with 100 mL of 5 mg/mL Ipamorelin (API) stock solution into 200 mL of Milli-Q water. At near complete volume, the initial pH of 5.18 was adjusted to 5.01 with glacial acetic acid.

### DP#4: 1.0 mg/mL Ipamorelin, 10 mM sodium acetate, pH 6, and 9mg/mL NaCl

900 mL of the formulation was prepared by mixing 100 mL of 10x sodium acetate pH ~5 stock solution with 500 mL NaCl stock solution with 200 mL of 5 mg/mL Ipamorelin (API) stock solution into 100 mL of Milli-Q water. At near complete volume, the initial pH of 6.79 was adjusted to 5.96 with glacial acetic acid.

### DP#5: 0.5 mg/mL Ipamorelin, 10 mM sodium citrate, pH 5, and 9mg/mL NaCl

900 mL of the formulation was prepared by mixing 100 mL of 10x sodium citrate pH ~5 stock solution with 500 mL NaCl stock solution with 100 mL of 5 mg/mL Ipamorelin (API) stock solution into 200 mL of Milli-Q water. At near complete volume, the initial pH of 4.75 was adjusted to 5.00 using IN sodium hydroxide.

### DP#8: 1.0 mg/mL Ipamorelin, 10 mM sodium citrate, pH 6, and 9mg/mL NaCl

900 mL of the formulation was prepared by mixing 100 mL of 10x sodium acetate pH ~6 stock solution with 500 mL NaCl stock solution with 200 mL of 5 mg/mL Ipamorelin (API) stock solution into 100 mL of Milli-Q water. At near complete volume, the initial pH of 5.67 was adjusted to 6.02 using IN sodium hydroxide.

### DP#10: 1.0 mg/mL Ipamorelin, 10 mM sodium citrate, pH 7, and 9mg/mL NaCl

900 mL of the formulation was prepared by mixing 100 mL of 10x sodium acetate pH ~7 stock solution with 500 mL NaCl stock solution with 200 mL of 5 mg/mL Ipamorelin (API) stock solution into 100 mL of Milli-Q water. At near complete volume, the initial pH of 7.16 was adjusted to 7.02 with IN hydrochloric acid.

### DP#15: 0.5 mg/mL Ipamorelin, 10 mM sodium phosphate, 2 mM sodium EDTA, pH 6 and 9 mg/ml NaCl

900 mL of the formulation was prepared by mixing 100 mL of 10x sodium phosphate, EDTA pH ~6 stock solution with 500 mL NaCl stock solution with 100 mL of 5 mg/mL Ipamorelin (API) stock solution into 200 mL of Milli-Q water. At near complete volume, the initial pH of 6.06 was adjusted to 6.00 with IN hydrochloric acid.

**Table 1. Summary of pH Results after 6 months stability testing**

| | Formulations | Temp | pH |
|---|---|---|---|
| C | 0.5 mg/mL Ipamorelin, 2 equivalents of acetic acid, pH 7.5, and 9 mg/mL NaCl | 40 °C | 7.5 |
| 1 | 0.5 mg/mL Ipamorelin, 10 mM Sodium acetate, pH 5, and 9 mg/mL NaCl | 40 °C | 5.0 |
| 4 | 1.0 mg/mL Ipamorelin, 10 mM Sodium acetate, pH 6, and 9 mg/mL NaCl | 40 °C | 6.0 |
| 5 | 0.5 mg/mL Ipamorelin, 10 mM Sodium citrate, pH 5, and 9 mg/mL NaCl | 40 °C | 5.0 |
| 8 | 1.0 mg/mL Ipamorelin, 10 mM Sodium citrate, pH 6, and 9 mg/mL NaCl | 40 °C | 6.1 |
| 10 | 1.0 mg/mL Ipamorelin, 10 mM Sodium citrate, pH 7, and 9 mg/mL NaCl | 40 °C | 7.1 |
| 15 | 0.5 mg/mL Ipamorelin, 10 mM sodium phosphate, 2 mM sodium EDTA, pH 6 and 9 mg/ml NaCl | 40 °C | 6.0 |

**TABLE 2. Summary of potency and overall purity results**

| | Theoretical mg/mL | Potency (mg/mL) | % Recovery | % Purity (Determined by Empower) |
|---|---|---|---|---|
| Control 40 C | 0.5 | 0.449 | 90 | 92.28 |
| DP#1 40C | 0.5 | 0.470 | 94 | 98.68 |
| DP#4 40C | 1.0 | 0.945 | 94 | 97.96 |
| DP#5 40C | 0.5 | 0.473 | 95 | 98.01 |
| DP#8 40C | 1.0 | 0.943 | 94 | 97.39 |
| DP# 10 40C | 1.0 | 0.899 | 90 | 93.06 |
| DP# 15 40C | 0.5 | 0.460 | 92 | 95.31 |

### EXAMPLE 2: Solubility as a Function of pH

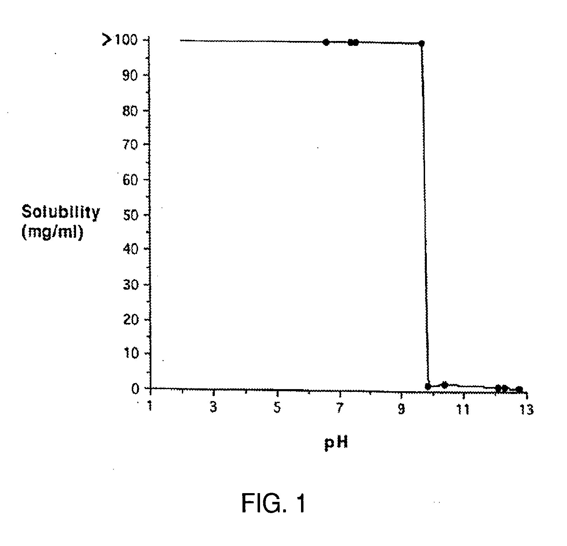

The solubility of ipamorelin free base at different pH values is shown graphically in Figure 1. At pH values less than approximately 10, the drug is very soluble in aqueous media (>100 mg/ml).

This dramatic change in solubility around pH 10 is reflected in the solubilities of ipamorelin free base and its diacetate salt in pure water. The diacetate salt dissolves readily: it is capable of producing solutions greater than 100 mg/ml, with a final pH of about 7.5. The free base is much less soluble; only about 1.5 mg/ml was measured in a saturated solution, pH = 9.83. However, solutions of much higher concentration can be achieved by simply adjusting the pH to around neutrality, using a solution of HCl or a similar acid.

### EXAMPLE 3: X-ray Diffraction (XRD)

XRD experiments of crystalline forms of ipamorelin free base at room temperature are illustrated in Figures 2 and 3. The experiments were conducted on a Scintag XDS 2000 powder X-ray diffractometer, using the following conditions for measurement:

| | |
|---|---|
| Goniometer type: | Theta/Theta |
| Goniometer radius: | 250 mm |
| Detector: | Liquid nitrogen cooled energy dispersive Ge detector |
| Voltage: | 45 KV |
| Current: | 40 mA |
| Filter: | No filter set |
| Radiation: | CuK_{*a*1} radiation (λ = 1.540598Å) |
| Target size: | 1.0 x 10 mm |
| Continuous scan, step size: | 0.02° |
| Speed: | 1°/min. |
| Divergent Beam-Slit: | 2 mm |
| Receiving-Slit: | 0.2 mm |
| Divergent Beam Scatter-Slit: | 4 mm |
| Scatter-Slit: | 0.5 mm |

The XRD profiles were measured at room temperature. No sample spinner was used. All diffractograms were measured from 2 to 40 degrees two-Theta.

For hot stage XRD experiments, approximately 40 mg of material was placed onto a sample holder (20 mm diameter x 0.2 mm deep), which was fastened to a variable temperature unit on the Scintag XDS 2000 powder X-ray diffractometer. The temperature was raised or lowered at a rate of 1°C/min pausing at pre-selected temperatures to allow diffractogram acquisition. Diffractograms were measured from 2 to 30 degrees two-Theta.

## Claims

1. An injection or infusion solution of ipamorelin comprising:
a) ipamorelin solubilized by two molar equivalents of acetic acid (ipamorelin diacetate), in an amount of from 0.001 to 20%, based on the weight of the free base of ipamorelin;
b) a molar excess of acid, preferably acetic acid, in an amount sufficient to give a pH of at least 3 and less than 7;
c) optionally from 0.1 to 30% of one or more formulation aids; and
d) water q.s. to 100%; all percentages based on the total weight of the solution.

2. The solution of claim 1 comprising from 1 to 10 molar equivalents of excess acetic acid.

3. The solution of claim 1, comprising excess phosphoric acid, citric acid, tartaric acid or lactic acid.

4. The solution of claims 1, 2 or 3, at a pH of from 4 to 6.5.

5. The solution of claim 1, 2, 3 or 4, in the absence of any solvent residue from the precipitation of ipamorelin diacetate.

6. The solution of claims 1, 2, 3, 4 or 5, wherein said ipamorelin diacetate is prepared by a process comprising mixing ipamorelin and acetic acid and water, at a molar ratio of 1:2 ipamorelin:acetic acid and an ipamorelin concentration of from 0.1 to 100 mg/ml.

7. The solution of claims 1, 2, 3, 4, 5 or 6, wherein said ipamorelin diacetate is prepared by a process comprising mixing ipamorelin and acetic acid and water, at a molar ratio of.1;2 ipamorelin:acetic acid and an ipamorelin concentration of from 1 to 10 mg/ml, and said ipamorelin is a free base having a dihydrate crystalline form.

8. The solution of claims 1, 2, 3, 4, 5, or 6, wherein said ipamorelin diacetate is prepared by a process comprising mixing ipamorelin and acetic acid and water, at a molar ratio of 1:2 ipamorelin:acetic acid and an ipamorelin concentration of from 1 to 10 mg/ml, and said ipamorelin is a free base having crystalline form A.

9. The solution of claims 1, 2, 3, 4, 5, 6, 7 or 8, wherein a pH buffering system is created by the inclusion of an alkaline salt of a suitable organic acid.

10. A process of producing injection and infusion solutions of ipamorelin diacetate, at a concentration of ipamorelin of from 0.001 to 20 %, without isolating the diacetate salt after formation, comprising:
a) mixing ipamorelin free base and acetic acid at a molar ratio of 1:2 to form an aqueous solution of ipamorelin diacetate;
b) mixing said ipamorelin diacetate with from 0.1 to 30% of one or more formulation aids, without isolating said ipamorelin diacetate in solid form; and
c) optionally mixing said ipamorelin diacetate with additional water so that the final concentration of ipamorelin is from 0.001 to 20%, based on the total weight of the solution and the weight of the free base of ipamorelin;
wherein steps (a), (b) and (c) are performed simultaneously, sequentially or in any order, and wherein said formulation aids comprise a pH adjusting agent adequale to adjust the pH of the solution to at least 3 and less than 7.

11. The process of claim 10, wherein said formulation aids comprise from 1 to 10 molar equivalents of excess acetic or citric acid, relative to said ipamorelin diacetate,

12. The process of claims 10 or 11, wherein said one or more formulation aids comprises a tonicifying effective amount of one or more tonicifying agents,

13. The process of claims 10, 11 or 12, wherein said ipamorelin in step (a) is a free base In a hydrated crystalline form comprising from 1.5 to 2.5 moles of water per mole of ipamorelin.

14. The process of claims 10, 11 or 12, wherein said ipamorelin in step (a) is a free base as crystalline form A.

## Patentansprüche

1. Injektions-oder Infusionslösung von Ipamorelin umfassend:
a) Ipamorelin gelöst in zwei-molaren Äquivalenten der Essigsäure (Ipamorelin Diacetat), in einer Menge von 0.001 bis 20%, bezogen auf das Gewicht der freien Base von Ipamorelin;
b) molarer Säure-Überschuss, bevorzugt Essigsäure, in einer Menge, die ausreicht, um einen pH-Wert von mindestens 3 und weniger als 7 zu erreichen;
c) optional von 0.1 bis 30% von einer oder mehreren Formulierungshilfsmitteln; und
d) Wasser q.s. bis 100%; alle Prozentangaben bezogen auf das Gesamtgewicht der Lösung.

2. Lösung nach Anspruch 1, umfassend 1 bis 10 molare Äquivalente von überschüssiger Essigsäure.

3. Lösung nach Anspruch 1, umfassend überschüssige Phosphorsäure, Zitronensäure, Weinsäure oder Milchsäure.

4. Lösung nach Anspruch 1, 2, oder 3 bei einem pH-Wert von 4 bis 6.5.

5. Lösung nach Anspruch 1, 2, 3 oder 4 in der abwesenheit eines Lösungsmittelrückstandes von der Ausfällung von Ipamorelin Diacetat.

6. Lösung nach Anspruch 1, 2, 3, 4 oder 5 wobei das Ipamorelin Diacetat durch ein Verfahren hergestellt wird, umfassend Mischen von Ipamorelin und Essigsäure und Wasser, in einem molaren Verhältnis von 1:2 Ipamorelin:Essigsäure und einer Ipamorelin-Konzentration von 0.1 bis 100 mg/ml.

7. Lösung nach Anspruch 1, 2, 3, 4, 5 oder 6, wobei das Ipamorelin Diacetat durch ein Verfahren hergestellt wird, umfassend Mischen von Ipamorelin und Essigsäure und Wasser, in einem molaren Verhältnis von 1:2 Ipamorelin:Essigsäure und einer Ipamorelin-Konzentration von 1 bis 10 mg/ml, und das Ipamorelin ist eine freie Base in einer Dihydrat kristallinen Form.

8. Lösung nach Anspruch 1, 2, 3, 4, 5 oder 6, wobei das Ipamorelin Diacetat durch ein Verfahren hergestellt wird, umfassend Mischen von Ipamorelin und Essigsäure und Wasser, in einem molaren Verhältnis von 1:2 Ipamorelin:Essigsäure und einer Ipamorelin-Konzentration von 1 bis 10 mg/ml, und das Ipamorelin ist eine freie Base in kristalliner Form A.

9. Lösung nach Anspruch 1, 2, 3, 4, 5, 6, 7 oder 8, wobei ein pH-Puffer-System durch die Einbeziehung von einem alkalischen Salz einer geeigneten organischen Säure erzeugt wird.

10. Verfahren zur Herstellung von Injektions- und Infusionslösungen von Ipamorelin Diacetat, in einer Konzentration von Ipamorelin von 0.001 bis 20%, ohne Isolieren des Diacetatsalzes nach der Bildung, umfassend:
a) Mischen der Ipamorelin freien Base und Essigsäure in einem molaren Verhältnis von etwa 1:2, um eine wässrige Lösung von Ipamorelin Diacetat zu bilden;
b) Mischen des Ipamorelin Diacetat mit 0.1 bis 30% einer oder mehrerer Formulierungshilfsmitteln, ohne das Ipamorelin Diacetat in fester Form zu isolieren; und
c) optional Mischen des Ipamorelin Diacetat mit zusätzlichem Wasser, so dass die Endkonzentration des Ipamorelin von 0.001 bis 20% ist, bezogen auf das Gesamtgewicht der Lösung und das Gewicht der freien Basen des Ipamorelin; wobei die Schritte (a), (b) und (c) gleichzeitig, nacheinander oder in beliebiger Reihenfolge durchgeführt werden, und wobei die Formulierungshilfsmitteln ein pH-Einstellmittel umfassen, dass ausreicht um den pH der Lösung auf mindestens 3 und weniger als 7 einzustellen.

11. Verfahren nach Anspruch 10, wobei die Formulierungshilfsmitteln 1 bis 10 molare Äquivalente von überschüssiger Essigsäure oder Zitronensäure umfassen, bezogen auf das Ipamorelin Diacetat.

12. Verfahren nach Anspruch 10 oder 11, wobei ein oder mehrere Formulierungshilfsmitteln eine tonisierende wirksame Menge von einem oder mehreren tonisierenden Mitteln umfasst.

13. Verfahren nach Anspruch 10, 11 oder 12, wobei das Ipamorelin in Schritt (a) eine freie Base in einer hydratisierten kristallinen Form ist, umfassend von 1.5 bis 2.5 Mol Wasser pro Mol Ipamorelin.

14. Verfahren nach Anspruch 10, 11 oder 12, wobei das Ipamorelin in Schritt (a) eine freie Base in kristalliner Form A ist.

## Revendications

1. Solution d'injection ou de perfusion d'ipamoréline comprenant :
a) de l'ipamoréline solubilisée par deux équivalents molaires d'acide acétique (diacétate d'ipamoréline), en une quantité de 0,001 à 20 %, sur la base du poids de la base libre d'ipamoréline ;
b) un excès molaire d'acide, de préférence l'acide acétique, en une quantité suffisante pour obtenir un pH d'au moins 3 et inférieur à 7 ;
c) facultativement de 0,1 à 30 % d'un ou plusieurs adjuvants de formulation ; et
d) de l'eau q.s. pour 100 % ; tous les pourcentages étant basés sur le poids total de la solution.

2. Solution de la revendication 1 comprenant de 1 à 10 équivalents molaires d'acide acétique en excès.

3. Solution de la revendication 1, comprenant un excès d'acide phosphorique, d'acide citrique, d'acide tartrique ou d'acide lactique.

4. Solution des revendications 1, 2 ou 3, à un pH de 4 à 6,5.

5. Solution de la revendication 1, 2, 3 ou 4, en l'absence d'un résidu de solvant quelconque à partir de la précipitation de diacétate d'ipamoréline.

6. Solution des revendications 1, 2, 3, 4 ou 5, dans laquelle ledit diacétate d'ipamoréline est préparée par un procédé comprenant le mélange d'ipamoréline et d'acide acétique et d'eau, à un rapport molaire d'ipamoréline:acide acétique 1:2 et une concentration d'ipamoréline de 0,1 à 100 mg/ml.

7. Solution des revendications 1, 2, 3, 4, 5 ou 6, dans laquelle ledit diacétate d'ipamoréline est préparé par un procédé comprenant le mélange d'ipamoréline et d'acide acétique et d'eau, à un rapport molaire d'ipamoréline:acide acétique 1:2 et une concentration d'ipamoréline de 1 à 10 mg/ml, et ladite ipamoréline est une base libre ayant une forme cristalline de dihydrate.

8. Solution des revendications 1, 2, 3, 4, 5 ou 6, dans laquelle ledit diacétate d'ipamoréline est préparé par un procédé comprenant le mélange d'ipamoréline et d'acide acétique et d'eau, à un rapport molaire d'ipamoréline:acide acétique 1:2 et une concentration d'ipamoréline de 1 à 10 mg/ml, et ladite ipamoréline est une base libre ayant une forme cristalline A.

9. Solution des revendications, 1, 2, 3, 4, 5, 6, 7 ou 8, dans laquelle un système de tampon de pH est créé par l'inclusion d'un sel alcalin d'un acide organique adapté.

10. Procédé de production de solutions d'injection et de perfusion de diacétate d'ipamoréline, à une concentration d'ipamoréline de 0,001 à 20 %, sans isolement du sel de diacétate après formation, comprenant :
a) le mélange de base libre d'ipamoréline et d'acide acétique à un rapport molaire de 1:2 pour former une solution aqueuse de diacétate d'ipamoréline ;
b) le mélange dudit diacétate d'ipamoréline avec de 0,1 à 30 % d'un ou plusieurs adjuvants de formulation, sans isoler ledit diacétate d'ipamoréline sous forme solide ; et
c) facultativement le mélange dudit diacétate d'ipamoréline avec de l'eau additionnelle de sorte que la concentration finale d'ipamoréline soit de 0,001 à 20 %, sur la base du poids total de la solution et le poids de la base libre d'ipamoréline ; dans lequel les étapes (a), (b) et (c) sont conduites simultanément, séquentiellement ou dans un ordre quelconque, et dans lequel lesdits adjuvants de formulation comprennent un agencement d'ajustement de pH adéquat pour ajuster le pH de la solution à au moins 3 et inférieur à 7.

11. Procédé de la revendication 10, dans lequel lesdits adjuvants de formulation comprennent de 1 à 10 équivalents molaires d'acide acétique ou citrique en excès, par rapport audit diacétate d'ipamoréline.

12. Procédé des revendications 10 ou 11, dans lequel lesdits un ou plusieurs adjuvants de formulation comprennent une quantité tonifiante efficace d'un ou plusieurs agents tonifiants.

13. Procédé des revendications 10, 11 ou 12, dans lequel ladite ipamoréline dans l'étape (a) est une base libre sous une forme cristalline hydratée comprenant de 1,5 à 2,5 moles d'eau par mole d'ipamoréline.

14. Procédé des revendications 10, 11 ou 12, dans lequel ladite ipamoréline dans l'étape (a) est une base libre sous la forme cristalline A.
